# EUROPEAN PATENT SPECIFICATION

(11) **EP 3 603 525 B1**
(45) Date of publication and mention of the grant of the patent: **08.03.2023**
(21) Application number: 17901601.9
(22) Date of filing: 24.10.2017
(51) Int. Cl.: A61B 8/00

(54) **ULTRASONIC IMAGING DEVICE AND CONTROL METHOD THEREOF**
ULTRASCHALLBILDGEBUNGSVORRICHTUNG UND STEUERUNGSVERFAHREN DAFÜR
DISPOSITIF D'IMAGERIE ULTRASONORE ET PROCÉDÉ DE COMMANDE ASSOCIÉ

(30) Priority: 21.03.2017 KR 20170035064
(43) Date of publication of application: 05.02.2020
(73) Proprietor: Samsung Medison Co., Ltd., Hongcheon-gun, Gangwon-do 25108 (KR)
(72) Inventor: LEE, Seung-Ju, Seoul 05665 (KR); KIM, Chil-Su, Seoul 05304 (KR); HA, Kil Su, Yongin-si Gyeonggi-do 16808 (KR); HYUN, Dong Gyu, Gwangju-si Gyeonggi-do 12798 (KR)
(74) Representative: D'Halleweyn, Nele Veerle Trees Gertrudis
(86) International application number: PCT/KR2017/011779
(87) International publication number: WO 2018/174361

(56) References cited:
- JP-A- H08 182 673
- JP-A- 2007 014 485
- JP-A- 2014 057 631
- JP-A- 2014 104 110
- KR-A- 20140 129 776
- KR-A- 20150 004 490
- KR-A- 20150 134 299
- KR-A- 20160 069 327
- US-A1- 2010 049 050
- US-A1- 2012 179 037
- US-A1- 2013 165 796
- US-A1- 2016 262 726

## Description

### 1. Field

The present invention relates to an ultrasound imaging apparatus for generating an image inside an object using ultrasound.

### 2. Description of the Related Art

An ultrasound diagnostic apparatus is a device for transmitting ultrasound from a surface of a subject toward a target point of the inside of the subject, and obtaining tomograms of soft tissue or images of blood flow in an non-invasive manner by receiving the reflected echo ultrasound.

The ultrasound diagnostic apparatus is widely used due to being compact and inexpensive, and is able to display diagnostic images in real time, as compared to X-ray devices, Computerized Tomography (CT) scanners, Magnetic Resonance Imaging (MRI) devices, etc.

The ultrasound diagnostic apparatus includes a probe for transmitting ultrasound to the subject and receiving the echo ultrasound reflected from the subject to obtain an ultrasound image of the subject.

There may be multiple probes equipped in the ultrasound diagnostic apparatus, and each probe may be coupled with a port of the ultrasound diagnostic apparatus over a wireless communication network or via a cable. Each probe may be held in a holder equipped in the ultrasound diagnostic apparatus.

Typically, if a user selects one of the plurality of probes held in the holder, the user has to manually type in which port the selected probe corresponds to. For example, in a case where there are first and second holders equipped in the ultrasound diagnostic apparatus and the user selects the probe held in the second holder, the user has to type in whether the probe held in the second holder is coupled with a first port or a second port directly to the ultrasound diagnostic apparatus.

In addition, the user may use the ultrasound imaging apparatus to diagnose a patient as a whole. The ultrasound imaging apparatus may include a program for guiding the user to diagnose the patient as a whole. In addition, the program may include a sequential diagnosis step of diagnosing the patient. In proceeding with the diagnostic step, it has been inconvenient for the user to replace the ultrasonic probe for accurate diagnosis.

US 2010/049050 A1 discloses an ultrasound machine operated by executing a protocol. Executing the protocol configures the ultrasound machine, controls for operating the ultrasound machine and a display for the ultrasound machine. Protocols may be defined to provide streamlined configuration of controls for operating the ultrasound machine, and to provide instructions that guide a user's operation of the ultrasound machine.

JP 2014 104110 A discloses an ultrasonic diagnostic apparatus including: a plurality of ultrasonic probes connected to the apparatus and gripped with a gripping part; a plurality of connector parts for obtaining signals used for generating an ultrasonic image via the connected ultrasonic probes; a control part for controlling selection of which connector part of the plurality of connectors to be in an active condition where the ultrasonic signal can be sent or received; and a detection part which detects the ultrasonic probe connected to the connector part in the active condition on the basis of the control, and which is provided in the vicinity of the gripping part.

### SUMMARY

Therefore, it is an aspect of the disclosure to provide an ultrasound imaging apparatus and a control method thereof which can be replaced more quickly and conveniently in replacing an ultrasound probe when a patient is diagnosed as a whole using the ultrasound imaging apparatus.

In accordance with the invention, an ultrasound imaging apparatus is defined in claim 1. controller may be configured to turn on the first light source or the second light source based on the program.

The ultrasound imaging apparatus not falling within the scope of the invention, further includes: a display unit to display the ultrasound image acquired by at least one of the first ultrasound probe and the second ultrasound probe, and the controller is configured to guide to replace the first ultrasound probe with the second ultrasound probe through the display unit based on the program.

The ultrasound imaging apparatus further includes: an input unit to receive a command from a user, and the controller is configured to store diagnosis contents of the first diagnosis step in the storage when the user inputs a save command through the input unit and guide to replace with the second ultrasound probe corresponding to the second diagnosis step.

The input unit receives information of the object from the user, and the controller is configured to guide to replace the first ultrasound probe with the second ultrasound probe by using the program and the information of the object.

The input unit receives information related to the ultrasound image from the user, and the controller is configured to guide to replace the first ultrasound probe with the second ultrasound probe by using the program and the information related to the ultrasound image.

The storage stores a set value of each of the first ultrasound probe and the second ultrasound probe when the ultrasound image is acquired, and the controller is configured to guide to replace with the second ultrasound probe corresponding to the second diagnosis step by applying the set value when changing from the first diagnosis step to the second diagnosis step based on the program.

Each of the first ultrasound probe and the second ultrasound probe is provided as one of a convex array probe, a linear array probe and a phased array probe.

The second ultrasound probe acquires an ultrasound image by using an ultrasound of a frequency band different from the first ultrasound probe.

In accordance with the invention, a control method of an ultrasound imaging apparatus is defined in claim 9.

The control method may further include: turning on a first light source corresponding to the first ultrasound probe or a second light source corresponding to the second ultrasound probe based on the program.

The guiding to replace with the second ultrasound probe - not falling within the scope of the invention - includes: guiding to replace the first ultrasound probe with the second ultrasound probe through a display unit based on the program.

The guiding to replace with the second ultrasound probe includes: storing diagnosis contents of the first diagnosis step in the storage when a user inputs a save command, and guiding to replace with the second ultrasound probe corresponding to the second diagnosis step.

The control method further includes: receiving information of an object from the user, the guiding to replace with the second ultrasound probe includes: guiding to replace the first ultrasound probe with the second ultrasound probe by using the program and the information of the object.

The guiding to replace with the second ultrasound probe includes: guiding to replace the first ultrasound probe with the second ultrasound probe by using the program and the information related to the ultrasound image.

The guiding to replace with the second ultrasound probe includes: guiding to replace with the second ultrasound probe corresponding to the second diagnosis step by applying the set value when changing from the first diagnosis step to the second diagnosis step based on the program.

### BRIEF DESCRIPTION OF THE DRAWINGS

FIG. 1 is an exterior view of an ultrasound diagnostic apparatus according to an embodiment of the present disclosure.
FIG. 2 is an exterior view of a probe.
FIG. 3 shows various probes in different shapes.
FIG. 4 is an enlarged view of holders equipped in a main body.
FIG. 5 is a perspective view illustrating a form in which an ultrasonic probe is held in a holder according to an exemplary embodiment.
FIG. 6 is a control block diagram of an ultrasound imaging apparatus according to an exemplary embodiment.
FIG. 7 is a control block diagram of a main body according to an embodiment of the present disclosure.
FIG. 8 is a diagram for explaining guiding a replacement of an ultrasonic probe by using a holder according to an exemplary embodiment.
FIG. 9 is a diagram for explaining guiding a replacement of an ultrasonic probe.
FIG. 10 is a diagram illustrating guiding an ultrasound probe using a display unit not falling within the scope of the invention.
FIG. 11 is a flowchart according to an exemplary embodiment.
FIG. 12 is a flowchart illustrating a general method for controlling an ultrasound diagnostic apparatus with a probe held in a holder according to an embodiment of the present disclosure.
FIG. 13 is a flowchart illustrating a general method for controlling an ultrasound diagnostic apparatus with a probe held out of a holder according to an embodiment of the present disclosure.

### DETAILED DESCRIPTION

The present disclosure will now be described more fully with reference to the accompanying drawings, in which exemplary embodiments of the disclosure are shown. The disclosure may, however, be embodied in many different forms and should not be construed as being limited to the embodiments set forth herein; rather, these embodiments are provided so that this disclosure will be thorough and complete, and will fully convey the concept of the disclosure to those skilled in the art. Like reference numerals in the drawings denote like elements, and thus their description will be omitted. In the description of the present disclosure, if it is determined that a detailed description of commonly-used technologies or structures related to the embodiments of the present disclosure may unnecessarily obscure the subject matter of the invention, the detailed description will be omitted. It will be understood that, although the terms first, second, third, etc., may be used herein to describe various elements, components, regions, layers and/or sections, these elements, components, regions, layers and/or sections should not be limited by these terms. These terms are only used to distinguish one element, component, region, layer or section from another region, layer or section.

Embodiments of an ultrasound diagnostic apparatus and method for controlling the same will be described in detail with reference to accompanying drawings.

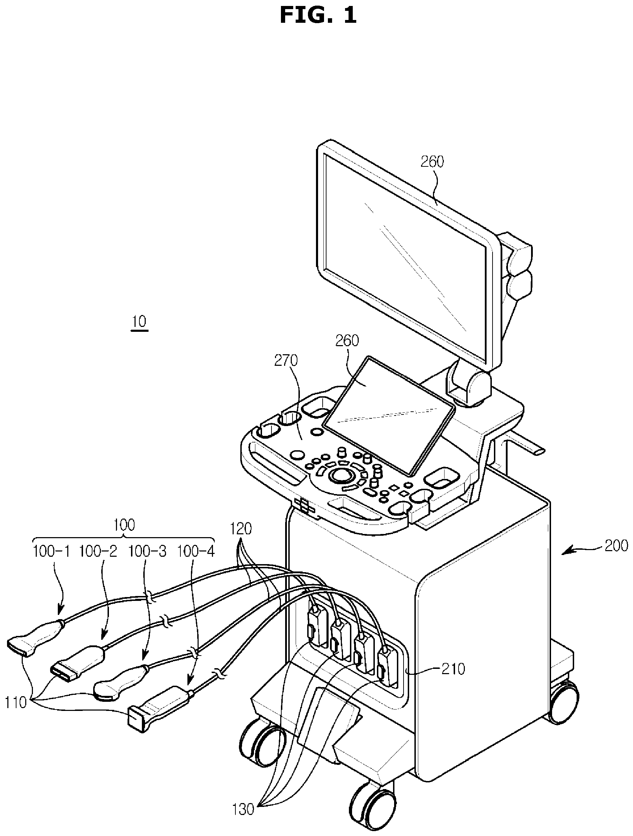

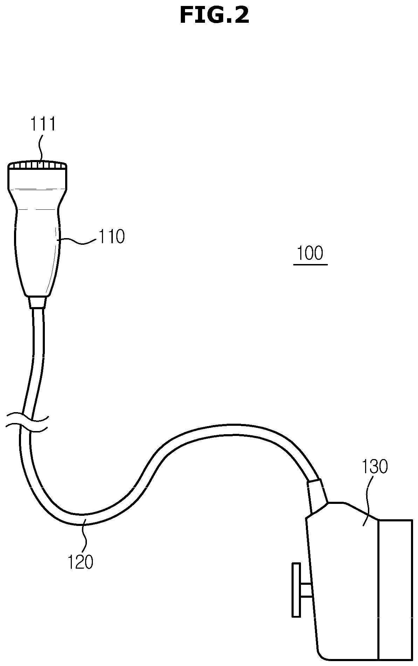

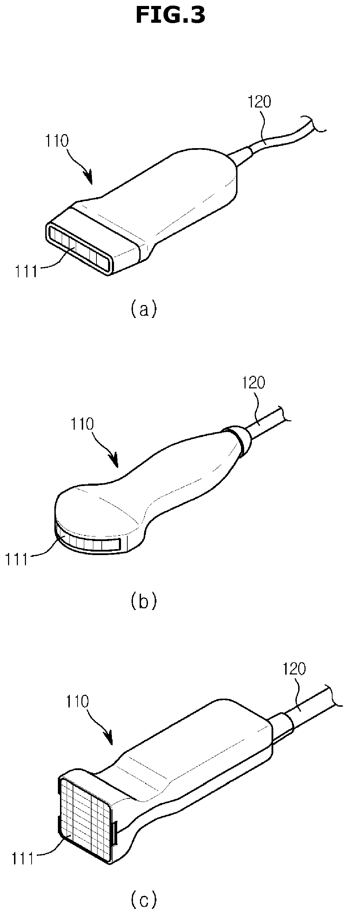

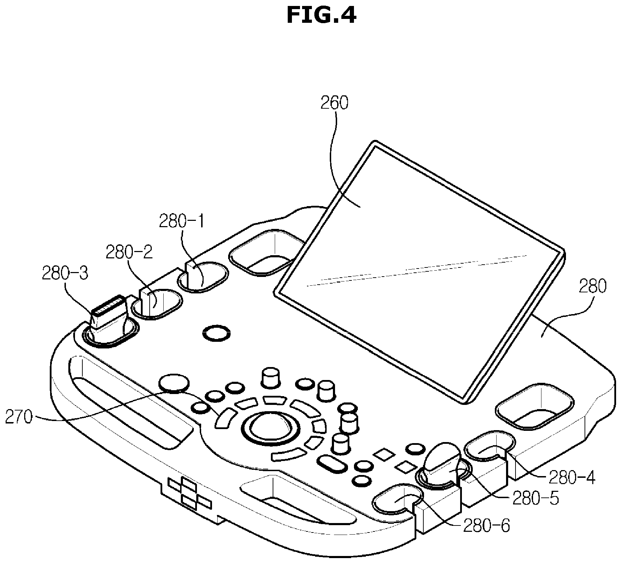

FIG. 1 is an exterior view of an ultrasound diagnostic apparatus according to an embodiment of the present disclosure, FIG. 2 is an exterior view of a probe, FIG. 3 shows various probes in different shapes, and FIG. 4 is an enlarged view of a plurality of holders equipped in a main body.

Referring to FIG. 1, an ultrasound diagnostic apparatus 10 may include an ultrasound probe 100 and a main body 200. The ultrasound probe 100 shown in FIG. 2 may be one of a plurality of ultrasound probes 100-1 to 100-4 shown in FIG. 1.

The ultrasound probe 100 may transmit ultrasound to a subject, and receive and convert the echo ultrasound reflected from the subject to an electric signal (hereinafter, referred to as an ultrasound signal).

The main body 200 may be coupled with the plurality of ultrasound probes 100-1 to 100-4 through at least one port 210. A first ultrasound probe and a second ultrasound probe are used to distinguish one ultrasound probe from another ultrasound probe, and the ultrasound probe is not limited by the above terms.

The main body 200 may create an ultrasound image based on the ultrasound signal received from the plurality of ultrasound probes 100-1 to 100-4.

The main body 200 may be a workstation having a display unit 260 and an input unit 270, and may be coupled with the plurality of ultrasound probes 100-1 to 100-4.

There may be a plurality of the display units 260 and the input units 270 equipped in the main body 200.

Features and operation methods of the plurality of ultrasound probes 100-1 to 100-4 will be described in detail in connection with FIG. 2. For convenience of explanation, the ultrasound probe 100 refers to each of the plurality of ultrasound probes 100-1 to 100-4, and may be one of the plurality of ultrasound probes 100-1 to 100-4.

The ultrasound probe 100 may include a transducer 110 for transmitting/receiving ultrasound, a probe connector 130 for transmitting/receiving signals to/from the main body 200, and a cable 120 connecting the transducer 110 and the probe connector 130.

The transducer 110 may transmit/receive ultrasound to/from the subject to obtain the ultrasound image of the inside of the subject.

Specifically, the transducer 110 may include a transducer module 111 for converting between electric signals and vibration (or acoustic) energy, which may use vibrators, such as piezoelectrics (not shown) to transmit ultrasound to the subject and receive the echo ultrasound reflected from the subject.

If the number of the vibrators is 64 to 256, coupling elements as many as the number of the vibrators are required in coupling the ultrasound probe 100 and the main body 200.

The object may be, but not exclusively, a living body of a human or animal, an organ in the living body, such as blood vessels, bones, muscles, etc., or anything whose internal structure may be imaged by the ultrasound diagnostic apparatus 10.

Referring to FIG. 3, the transducer 110 may be implemented as a linear transducer having a linear surface as shown in (a) of FIG. 3, as a convex transducer having a convex and curved surface as shown in (b) of FIG. 3, or as a matrix transducer as shown in (c) of FIG. 3, depending on an arrangement form of the transducer module 111. However, the transducer 110 is not limited thereto, and may be implemented in any other form than those shown in FIG. 3, which is known to a person having ordinary skill in the art, such as a phased array transducer.

The transducer 110 is connected to an end of the cable 120, the other end of which may be connected to the probe connector 130.

The ultrasound probe connector 130 is connected to a port of the main body 200 for transmitting/receiving electric signals with the main body 200.

The ultrasound probe connector 130 may be implemented as a connector combined with the port of the main body 200 implemented as a female connector.

Referring to FIG. 4, a holder 280 may be arranged in the main body 200 to hold the ultrasound probe 100. There may be a plurality of the holders 280 arranged independently of the number of the ultrasound probes 100 or the number of the ports 210.

The holder 280 provided in the main body 200 may be provided as first to sixth holders 280-1 to 280-6. An object sensor and an electromagnetic wave sensor may be provided in the first to sixth holders 280-1 to 280-6, respectively. In this case, the object sensor and the electromagnetic sensor may be provided to correspond to the number of the holders 280, may be provided on the holder 280 or around the holder 280. The object sensor senses the physical change around the holder 280. For example, the object sensor may be a conductor sensor, an infrared sensor, an LED sensor, or a magnetic sensor.

On the other hand, the holder is provided with a light source. When the light source guides the replacement of the first ultrasound probe with the second ultrasound probe as described below, the light source can illuminate the light so that the user can intuitively recognize the holder on which the second ultrasound probe is held. The light source may be composed of LEDs, but the type is not limited as long as the light source can be irradiated.

When the object sensor is the conductor sensor, the impedance or resonance frequency detected by the conductor sensor may vary depending on whether the ultrasound probe 100 is held in the holder 280. In this case, an object sensor sensing value processor may determine whether the ultrasound probe is held in the basis of the impedance or resonance frequency detected by the conductor sensor, that is, the sensing value.

As shown in FIG. 4, the first to sixth holders 280-1 to 280-6 may be provided around the display unit 260 or the input unit 270 of the main body 200. However, the position provided is not necessarily limited thereto.

In FIG. 4, the ultrasound probe is held in the third holder 280-3 and the fifth holder 280-5, and the light source irradiates light to the corresponding holder. However, the position of the ultrasound probe is not limited thereto. The ultrasound probe may be held in all of the holders 280-1 to 280-6.

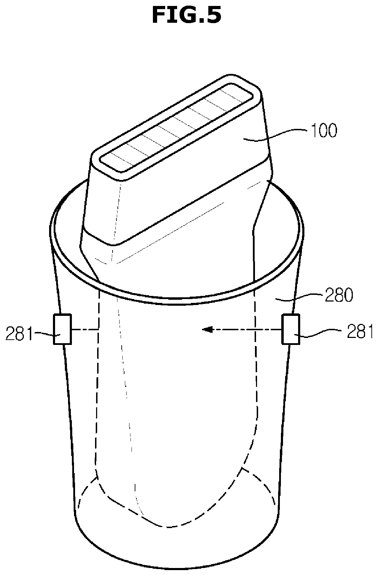

FIG. 5 is a perspective view illustrating a form in which an ultrasonic probe is held in a holder according to an exemplary embodiment. Referring to FIG. 5, each of the holders 280 may be provided with an object sensor 282 and a light source 281. The object sensor 282 may detect a situation in which the ultrasound probe 100 is held or held out. The object sensor 282 may be implemented as an infrared sensor, a magnetic sensor, a capacitance sensor, and an electromagnetic wave sensor, and the type of sensor is not limited as long as it is a sensor capable of acquiring a holding state of the ultrasound probe 100.

Meanwhile, the holder 280 has the light source 281. The light source 281 irradiates light for guiding the ultrasound probe to be used based on a diagnosis step of a program and the user's command as described below. Specifically, a first light source 281-1 is provided in the holder 280 on which the first ultrasound probe is held, and a second light source 281-2 is provided in the holder 280 on which the second ultrasound probe 100-2 is held.
Detailed operations related to the first light source and the second light source will be described later.

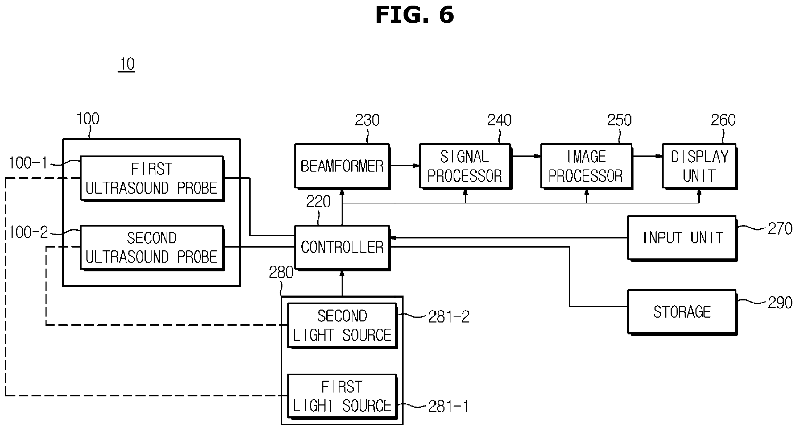

FIG. 6 is a control block diagram of an ultrasound imaging apparatus according to an exemplary embodiment.

Referring to FIG. 6, the ultrasound imaging apparatus includes a controller, a beamformer, a signal processor, an image processor, a display unit, an input unit, a holder, and a storage.

The controller 220 may generate control signals to control the respective elements of the ultrasound probe 100 and the main body 200.

For example, the controller 220 may generate control signals to operate the ultrasound probe 100, or generate control signals to control a beamformer 230, a signal processor 240, an image processor 250, and the display unit 260 based on ultrasound signals received from the ultrasound probe 100, and process various information obtained by the ultrasound probe 100 according to a stored program. The controller 220 and the ultrasound probe 100 may be connected through a port.

The port may be provided with a plurality of the ports. The plurality of ports provided with the main body 200 may be connected with the plurality of ultrasound probes.

A storage 290 may be implemented as at least one of a non-volatile memory device (for example, a cache, Read Only Memory (ROM), Programmable ROM (PROM), Erasable Programmable ROM (EPROM), Electrically Erasable Programmable ROM (EEPROM), and flash memory), a volatile memory device (for example, Random Access Memory (RAM)), or a storage medium (for example, Hard Disk Drive (HDD) and Compact Disc Read Only Memory (CD-ROM)), although not limited to these. The storage 290 may be a memory implemented as a separate chip from the processor described above with respect to the controller 220, or may be implemented as a single chip with the processor.

The storage 290 may store a program for guiding the diagnosis of the patient. Specifically, the user may diagnose the entire area, not just a specific area, using the ultrasound imaging apparatus. In this case, the program stored in the storage may indicate the step of diagnosing the patient on the display unit for fast and accurate diagnosis of the patient. That is, the user can guide the first diagnosis step for diagnosing the patient and then guide the second diagnosis step. For example, the program can guide the first diagnosis step to diagnose the liver before the user diagnoses the patient and then to the second diagnosis step to diagnose the kidneys. In addition, the program may guide by displaying the ultrasound image of the area being diagnosed by the user on the display unit and by displaying an ultrasound image mode, annotation, display layout, image parameter, and a preset of the ultrasound probe.

On the other hand, the storage 290 may store information of the patient obtained by using the ultrasound probe 100 during each diagnosis step. The patient information may include a set value of the ultrasound probe 100 set to acquire the ultrasound image and the ultrasound image acquired by the ultrasound probe 100.

The controller 220 may include a memory for storing a program and data, e.g., mapping table, to control the respective elements of the ultrasound probe 100 and the main body 200, and a processor for controlling the ultrasound probe 100 and the main body 200 according to the program and data stored in the memory.

In addition, the controller 220 may guide to replace a probe suitable for each diagnosis step when the diagnosis step proceeds, that is, when the diagnosis step proceeds from the first diagnosis step to the second diagnosis step based on the program stored in the storage 290. For example, when diagnosing the patient's liver in the first diagnosis step, the controller 220 may guide the user to use a convex ultrasound probe, and when diagnosing the patient's kidneys in the second diagnosis step after completing the first diagnosis step, the controller 220 may guide the user to use a matrix array ultrasound probe. There is no limitation on how the controller 220 guides the ultrasound probe used by the user, but according to the invention, the controller 220 is configured to control the light source provided in the holder to irradiate light to guide the user to intuitively use the ultrasound probe. The detailed description thereof will be described later.

The beamformer 230 is a device to provide a proper time delay to ultrasound for transmission or received echo ultrasound, in order for the ultrasound produced by the transducer of the ultrasound probe 100 to be focused on a target point of the subject at a desired moment of time, or for the echo ultrasound bounced off the target point of the subject to overcome a time difference in arriving to the transducer.

In the ultrasound diagnostic apparatus 10, the beamformer 230 may be included in the main body 200 that corresponds to a back-end as shown in FIG. 6, or may be included in the ultrasound probe 100 that corresponds to a front-end. Embodiments of the beamformer 230 are not limited thereto, and all or parts of elements of the beamformer 230 may be included in some parts of the front-end and back-end. However, for convenience of explanation, it is assumed that the beamformer 230 is included in the main body 200.

The signal processor 240 may convert a signal received from the beamformer 230 into a format suitable for image processing. For example, the signal processor 240 may perform filtering to eliminate noise outside a desired frequency band.

Furthermore, the signal processor 240 may be implemented by a digital signal processor (DSP), and may generate ultrasound image data by performing envelope detection to detect the amplitude of the echo ultrasound based on the signal received from the beamformer 230.

The image processor 250 may generate an image for the user, e.g., a doctor or patient, to visually check the inside of the subject, e.g., a human body, based on the ultrasound image data generated by the signal processor 240.

The image processor 250 may send the display unit 260 the ultrasound image generated using the ultrasound image data.

In addition, the image processor 250 may further perform extra image processing on the ultrasound image in some embodiments. For example, the image processor 250 may further perform post image processing, such as compensating for or readjusting contrast, brightness, or sharpness of the ultrasound image.

Such extra image processing of the image processor 250 may be performed according to a predetermined setting, or in response to a user instruction or command input through the input unit 270.

The display unit 260 may display the ultrasound image generated by the image processor 250, thereby enabling the user to visually examine the internal structure or tissue of the subject.

The display unit 260 may be implemented by various known display methods such as a cathode ray tube (CRT) and a liquid crystal display (LCD).

The display unit 260 displays an ultrasound image mode, annotation, display layout, image parameter, and preset of the ultrasound probe based on the program stored in the storage.

In addition, when the controller 220 guides the replacement of the first ultrasound probe 100-1 to the second ultrasound probe 100-2, the display unit 260 may display an image to be visually recognized by the user by displaying guide content. For example, the display unit 260 may display the position of each of the ultrasound probes 100 in the holder 280.

The input unit 270 may receive predetermined instructions or commands from the user for controlling the ultrasound diagnostic apparatus 10. The input unit 270 may also include a user interface, such as a keyboard, a mouse, a trackball, a touch screen, a paddle, etc.

For example, the input unit 270 may receive instructions to operate or freeze an operation of any one of the first ultrasound probe 100-1 and the second ultrasound probe 100-2.

In addition, the user may input a save command for storing medical contents corresponding to each diagnosis step through the input unit 270. Since the input of the save command through the input unit 270 means that one step of the diagnosis step of the program is completed, the controller 220 may store diagnosis contents in the storage 290, and at this time, the controller 220 may guide to replace the first ultrasound probe 100-1 with the second ultrasound probe 100-2.

The input unit 270 may receive information related to an object diagnosed by the user and the ultrasound image.

The object information includes a diagnosis site of the patient, a disease name to be diagnosed, a length of the object, a width of the object, and a volume of the object, and the controller may guide the probe to be changed based on the information.

The information related to the ultrasound image may include ROI of the object, depth of the ultrasound image, and resolution information of the ultrasound image.

The object information input by the user and information related to the ultrasound image may be used together with the program stored in the storage 290 to guide the replacement of the first ultrasound probe 100-1 with the second ultrasound probe 100-2. For example, if the disease of the patient to be measured by the user is cirrhosis of the patient, it is necessary to acquire a high resolution ultrasound image. Therefore, the user may be guided to replace with the linear array ultrasound probe.

The ultrasound probe 100 may be held in the holder 280.

When the main body 200 is connected to the plurality of ultrasound probes through the port 210, the plurality of ultrasound probes may be held in the holder 280. When the controller 220 guides to replace the first ultrasound probe 100-1 with the second ultrasound probe 100-2, the controller 220 controls the second light source 281-2 to irradiate light to guide the user to replace the first ultrasound probe 100-1 with the second ultrasound probe 100-2.

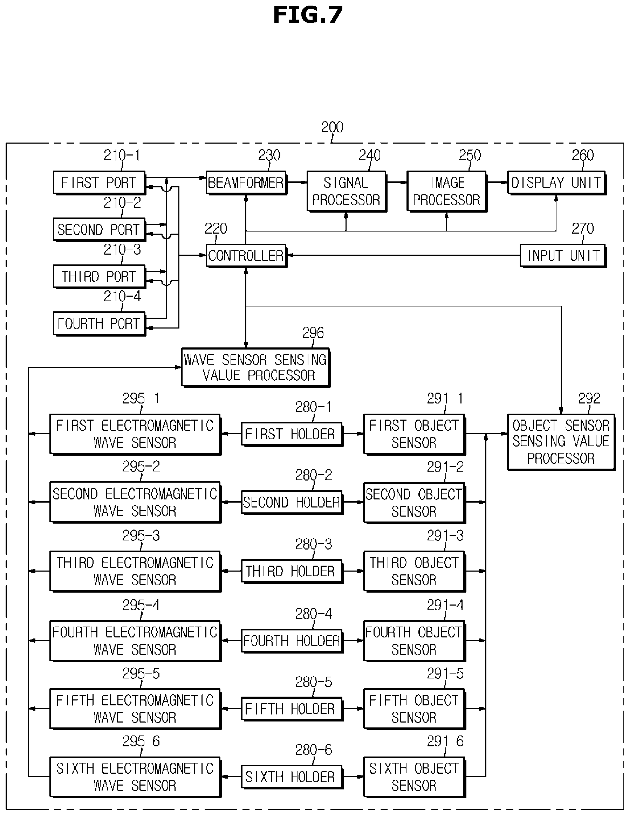

FIG. 7 is a control block diagram of a main body according to an embodiment of the present disclosure. Referring to FIG. 7, the first to sixth holders 280-1 to 280-6 may each be equipped with an object sensor 291 and an electromagnetic wave sensor 295. In this case, there may be the same number of the object sensors 291 and the electromagnetic wave sensors 295 as the number of the holders 280, and the object sensor 291 and the electromagnetic wave sensor 295 may be located on or around the holder 280.

The object sensor 291 may detect physical changes around the holder 280. For example, the object sensor 291 may be a conductor sensor, an infrared sensor, an Light Emitting Diode (LED) sensor, or a magnetic sensor.

If the object sensor 291 is the conductor sensor, the conductor sensor may detect a different impedance or a resonance frequency depending on whether the holder 280 holds the ultrasound probe 100. In this regard, an object sensor sensing value processor 292 may determine whether the ultrasound probe 100 is held in based on the impedance or resonance frequency, i.e., a sensing value detected by the conductor sensor.

The object sensor sensing value processor 292 may determine which one of the first to sixth holders 280-1 to 280-6 the ultrasound probe 100 is held in or held out, based on the sensing values from a plurality of object sensors 291-1 to 291-6.

The electromagnetic wave sensor 295 may detect electromagnetic waves produced around the holder 280. For example, the electromagnetic wave sensor 295 may be an Electro-Magnetic Interference (EMI) sensor.

In the case the electromagnetic wave sensor 295 is the EMI sensor, and the EMI sensor may detect different electromagnetic wave signals depending on whether the holder 280 holds the ultrasound probe 100 and whether the ultrasound probe 100 held in the holder 280 is activated. In this regard, an electromagnetic wave sensor sensing value processor 296 may determine whether the ultrasound probe 100 is activated based on the electromagnetic wave signals detected by the EMI sensor.

Specifically, if the ultrasound probe 100 is held in the holder 280 while being activated, the EMI sensor may detect electromagnetic waves, and otherwise if the ultrasound probe 100 held in the holder 280 is not activated, the EMI sensor may not detect any electromagnetic waves.

The electromagnetic wave sensor sensing value processor 296 may determine which one of the first to sixth holders 280-1 to 280-6 the ultrasound probe 100 is held in while being activated, based on the sensing values from a plurality of electromagnetic wave sensors 295-1 to 295-6.

In some embodiments, the controller 220, the object sensor sensing value processor 292, and the electromagnetic wave sensor sensing value processor 296 may be implemented to have separate memories and processors or implemented with a single memory and a single processor. If the object sensor sensing value processor 292 and the electromagnetic wave sensor sensing value processor 296 are implemented with a single memory and a single processor, a single sensing value processor may perform functions of the object sensor sensing value processor 292 and the electromagnetic wave sensor sensing value processor 296.

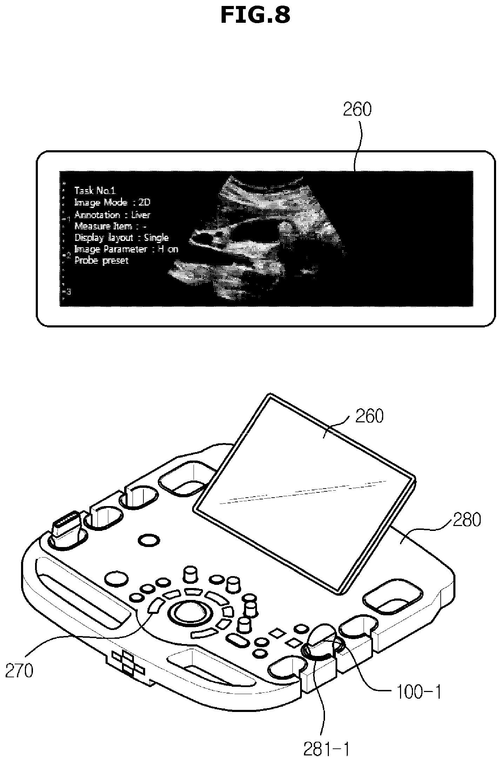

FIG. 8 is a diagram illustrating guiding an ultrasound probe using a holder according to an exemplary embodiment.

Referring to FIG. 8, FIG. 8 illustrates a diagnosis screen of the first diagnosis step of the program and the first ultrasound probe 100-1 corresponding thereto. The ultrasound image displayed on the display unit 260 displays the ultrasound image acquired by using the first ultrasound probe 100-1. At the same time, a diagnosis step (Task No.), an image mode, an annotation, a display layout, an image parameter, and a preset of the ultrasound probe may be displayed.

On the other hand, the controller 220 may control to irradiate light to the holder on which the first ultrasound probe 100-1 used in the first diagnosis step is held. The holder in which the first ultrasound probe 100-1 is held may include the first light source 281-1 corresponding to the first ultrasound probe 100-1. The controller 220 may control the first light source 281-1 to irradiate light to guide the user to use the first ultrasound probe 100-1 in treating the patient. For example, when the user diagnoses the patient's liver in the first diagnosis step by the stored program, the controller 220 may control the first light source 281-1 to irradiate light to the holder in which a convex array probe is held .

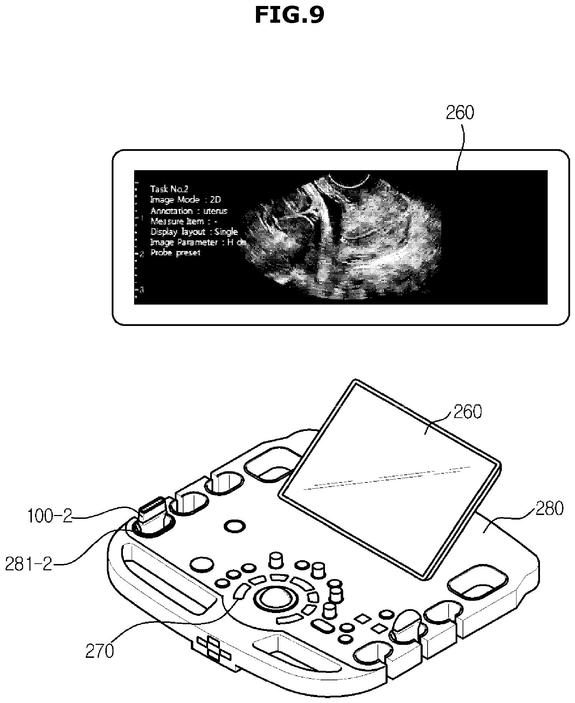

Referring to FIG. 9, FIG. 9 illustrates the diagnosis screen of the first diagnosis step of the program and the first ultrasound probe corresponding thereto. Referring to FIG. 9, the user may complete the first diagnosis step and proceed to the second diagnosis step. The user may input the save command through the input unit 270 to proceed from the first diagnosis step to the second diagnosis step. When the user inputs the save command through the input unit 270, the controller 220 may store a diagnostic record in which the user diagnoses the patient in the storage 290. The diagnostic record of the patient may include the ultrasound image obtained by using the ultrasound probe 100, a record of the patient and a setting state of the ultrasound. In this case, the stored ultrasound setting state may be used to set the first ultrasound probe 100-1 when the user performs the first diagnosis step later.

Meanwhile, when the user inputs the save command through the input unit 270, the second diagnosis step may be performed based on the program. When proceeding from the first diagnosis step to the second diagnosis step, the controller 220 controls the second light source 281-2 provided in the holder in which the second ultrasound probe 100-2 is held to irradiate light. The controller 220 controls the second light source 281-2 provided in the holder 280 held with the second ultrasound probe 100-2 to irradiate light, and the user can intuitively know the position of the second ultrasound probe 100-2 located in the holder 280. Therefore, the user can easily and quickly use the second ultrasound probe 100-2 to diagnose the patient.

The first ultrasound probe 100-1, the second ultrasound probe 100-2, the first diagnosis step, the second diagnosis step, the first light source 281-1, and the second light source 281-2 have been described for the purpose of distinguishing each configuration to explain the operation of the technology. The ultrasound probe, the contents of the diagnosis step and the implementation form of the light source are not limited.

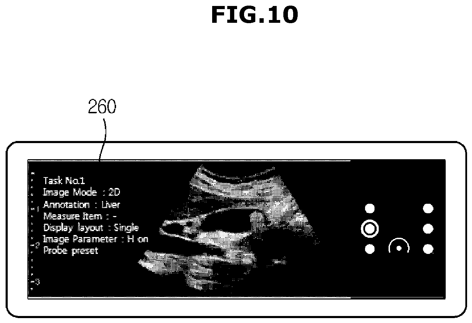

FIG. 10 is a diagram illustrating guiding an ultrasound probe using a display unit not falling within the scope of the invention.

Referring to FIG. 10, the display unit 260 shown in FIG. 10 schematically represents the holder. The schematic diagram of the holder displayed on the display unit 260 indicates where the first ultrasound probe 100-1 and the second ultrasound probe 100-2 are located. When the first diagnosis step is performed by the program stored in the storage 290, the controller 220 may display the position of the holder in which the first ultrasound probe 100-1 corresponding to the first diagnosis step is located, and the user may recognize this and diagnose the patient by using the first ultrasound probe 100-1. Similarly, when the user proceeds with the second diagnosis step, the controller may display the position of the holder in which the second ultrasound probe 100-2 corresponding to the second diagnosis step is located on the display unit. In FIG. 8, although the position of the ultrasound probe is shown in a schematic view of six holders provided on the right side of the ultrasound image, a method of displaying the position of the ultrasound probe is not limited.

Referring to FIGS. 8, 9 and 10, when guiding the first ultrasound probe and the second ultrasound probe by the light source provided in the holder, the user may intuitively use the ultrasound probe.

On the other hand, when displaying and guiding the location of the ultrasound probe on the display unit, the ultrasound image acquired using the ultrasound probe and the location of the ultrasound probe can be displayed on one screen.

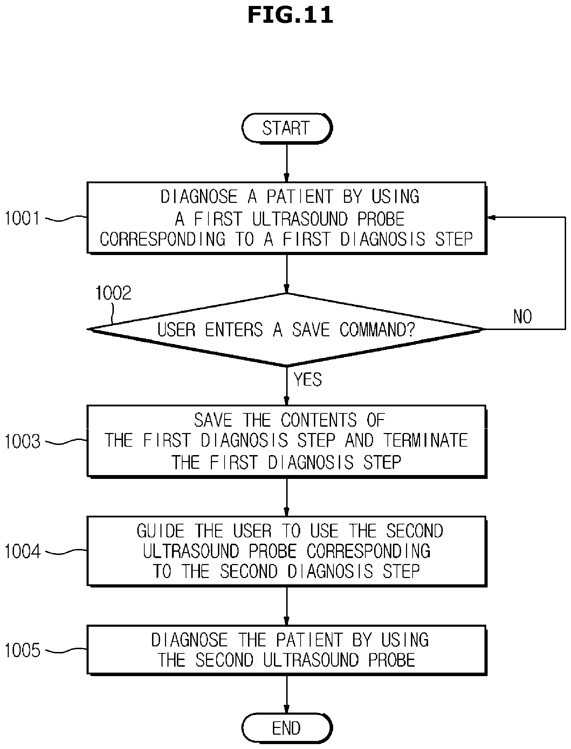

FIG. 11 is a flowchart according to an embodiment of the present invention.

Referring to FIG. 11, the user may proceed to the first diagnosis step of diagnosing the patient based on the program stored in the storage using the first ultrasound probe (1001). When the user completes the first diagnosis step and enters the save command, the controller saves the contents of the first diagnosis step in the storage and terminates the first diagnosis step (1003). After completing the first diagnosis step, the controller proceeds to the second diagnosis step. The controller may guide the user to use the second ultrasound probe corresponding to the second diagnosis step (1004). The controller guides the second ultrasound probe corresponding to the second diagnosis step through the light source provided in the ultrasound probe holder or through the display unit. The controller guides the user to use the second ultrasound probe corresponding to the second diagnosis step, and the user may diagnose the patient by proceeding with the second diagnosis step by using the second ultrasound probe (1005).

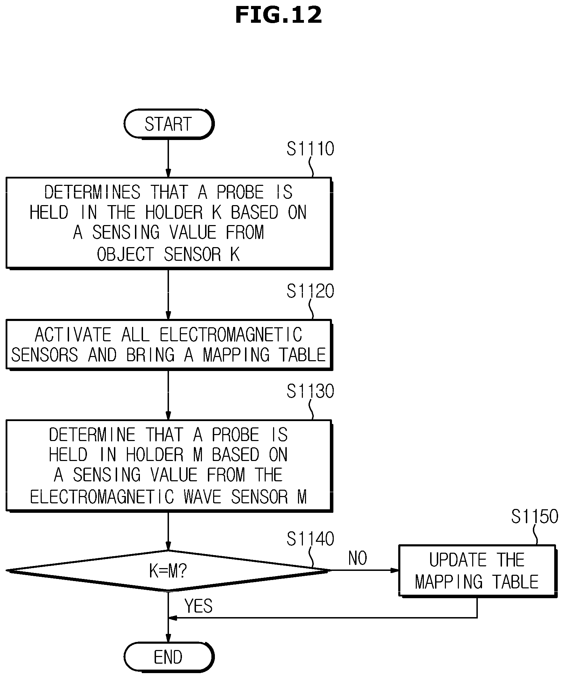

FIG. 12 is a flowchart illustrating a general method for controlling an ultrasound diagnostic apparatus with a probe held in a holder according to an embodiment of the present disclosure.

Referring to FIG. 12, if the ultrasound probe 100 is held in a holder k while the first to sixth object sensors 291-1 to 291-6 equipped in the first to sixth holders 280-1 to 280-6 are activated, the object sensor sensing value processor 292 determines that the ultrasound probe 100 is held in the holder k based on the sensing value from an object sensor k in operation (S1110).

The controller 220 may determine whether the ultrasound probe 100 held in is activated based on the sensing values from the first to sixth electromagnetic wave sensors 295-1 to 295-6 arranged in the first to sixth holders 280-1 to 280-6, and may update the mapping table.

Specifically, the controller 220 determines that the ultrasound probe 100 comes into contact with the object sensor k that corresponds to the holder k while the first to sixth object sensors 291-1 to 291-6 equipped in the first to sixth holders 280-1 to 280-6, respectively, are activated, i.e., the controller 220 determines that the ultrasound probe 100 is now held in the holder k in operation (S1110). In this case, the probe may detect an operation status. The controller 220 then brings the stored existing mapping table with reference to the sensing values from the first to sixth electromagnetic wave sensors 295-1 to 295-6 in operation (S1120).

If an electromagnetic wave sensor m detects activation of the ultrasound probe 100, the electromagnetic wave sensor sensing value processor 296 may determine that the currently activated probe is held in a holder m, based on the sensing value from the electromagnetic wave sensor m in operation (S1130).

Next, the controller 220 determines whether the holder k and the holder m correspond to each other in operation (S1140).

If the holder k and the holder m correspond to each other, the controller 220 keeps the mapping table intact, but otherwise, if the holder k and the holder m do not correspond to each other, the controller 220 updates the mapping table in operation (S1150).

Specifically, if the holder k and the holder m do not correspond, the controller 220 activates all ports 210-1 to 210-4 in a predetermined order, e.g., in a sequential order, and the electromagnetic wave sensor sensing value processor 296 examines each of the holders 280-1 to 280-6 corresponding to one of the ports 210-1 to 210-4 based on the sensing values collected from the first to sixth electromagnetic wave sensors 295-1 to 295-6. The controller 220 then newly updates the mapping table with a holder name corresponding to each port name.

For example, if the holder k and the holder m do not correspond, the controller 220 first activates the first port 210-1, activates the first to sixth electromagnetic wave sensors 295-1 to 295-6, and maps the second holder 280-2 and the first port 210-1 if an electromagnetic wave is detected by the second electromagnetic wave sensor 295-2 equipped in the second holder 280-2 when the ultrasound probe 100 is held in the second holder 280-2.

Next, the controller 220 activates the second port 210-2, activates the first to sixth electromagnetic wave sensors 295-1 to 295-6, and maps the fourth holder 280-4 and the second port 210-2 if an electromagnetic wave is detected by the fourth electromagnetic wave sensor 295-4 equipped in the fourth holder 280-4 when the ultrasound probe 100 is held in the fourth holder 280-4.

Subsequently, the controller 220 activates the third port 210-3, activates the first to sixth electromagnetic wave sensors 295-1 to 295-6, and maps the fifth holder 280-5 and the third port 210-3 if an electromagnetic wave is detected by the fifth electromagnetic wave sensor 295-5 equipped in the fifth holder 280-5 when the ultrasound probe 100 is held in the fifth holder 280-5.

Subsequently, the controller 220 activates the fourth port 210-4, activates the first to sixth electromagnetic wave sensors 295-1 to 295-6, and maps the first holder 280-1 and the fourth port 210-4 if an electromagnetic wave is detected by the first electromagnetic wave sensor 295-1 equipped in the first holder 280-1 when the ultrasound probe 100 is held in the first holder 280-1.

The updated mapping table or the port name of the ultrasound probe 100 held in the holder m may be indicated for the user through the display unit 260.

Furthermore, if it is determined that the plurality of ultrasound probes 100-1 to 100-6 are all held in any of the holders 280, the operation of all the ultrasound probes 100-1 to 100-6 may be automatically stopped (Auto Freeze).

While the previous embodiments show that the first to fourth ports 210-1 to 210-4 are mapped to the second, fourth, fifth, and first holders 280-2, 280-4, 280-5, and 280-1, respectively, the present disclosure is not limited thereto.

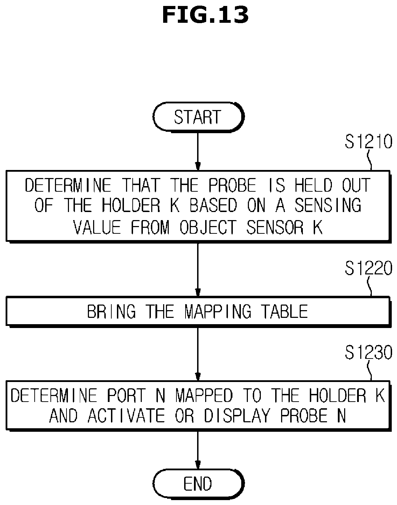

FIG. 13 is a flowchart illustrating a general method for controlling an ultrasound diagnostic apparatus with a probe held out of a holder according to an embodiment of the present disclosure.

First, if the ultrasound probe 100 is held out of the holder k while the first to sixth object sensors 291-1 to 291-6 equipped in the first to sixth holders 280-1 to 280-6 are activated, the object sensor sensing value processor 292 determines that the ultrasound probe 100 is held out of the holder k based on the sensing value from the object sensor k in operation (S1210).

Next, the controller 220 may determine a port n mapped to the holder k based on the stored mapping table in operation (S1220), and activate a probe n connected to the port n in operation (S1230).

Furthermore, the current mapping table, or the port number (n) and port name of the ultrasound probe 100 held out of the holder k may be indicated for the user through the display unit 260. In this case, the user may check the port name and manually type in whether the probe n is activated or not, through the input unit 270.

While the previous embodiments show that the plurality of ports 210-1 to 210-6 and the plurality of holders 280-1 to 280-6 are equipped in the single main body 200, the holders 280-1 to 280-6 may be equipped in separate devices and connected to the main body 200 over a wired/wireless communication network.

The ultrasound imaging apparatus and a control method thereof according to an embodiment can be replaced more quickly and conveniently in replacing the ultrasound probe when the patient is diagnosed as a whole using the ultrasound imaging apparatus.

Meanwhile, the aforementioned embodiments may be embodied in the form of a recording medium storing instructions executable by a computer. The instructions may be stored in the form of program codes and perform the operation of the disclosed embodiments by creating a program module when executed by a processor. The recording medium may be embodied as a computer readable recording medium.

The computer readable recording medium includes all types of recording media that store instructions readable by a computer such as read only memory (ROM), random access memory (RAM), a magnetic tape, a magnetic disc, a flash memory, and an optical data storage device.

Although a few embodiments of the present disclosure have been shown and described, it would be appreciated by those skilled in the art that changes may be made in these embodiments as far as they fall within the scope of the invention, which is defined in the claims.

## Claims

1. An ultrasound imaging apparatus (10) comprising:
a first ultrasound probe (100-1) configured to obtain an ultrasound image of an object by transmitting and receiving an ultrasound signal;
a second ultrasound probe (100-2) provided as a different type of ultrasound probe from the first ultrasound probe (100-1);
a storage (290) to store a program for guiding a diagnosis sequence of a patient comprising a predetermined first diagnosis step and a second diagnosis step;
a controller (220) configured to guide to diagnose the patient using the first ultrasound probe (100-1) corresponding to the first diagnosis step and guide to replace with the second ultrasound probe (100-2) corresponding to the second diagnosis step when changing from the first diagnosis step to the second diagnosis step based on the program; and
a holder (280) comprising a first light source (281-1) corresponding to the first ultrasound probe (100-1) and a second light source (281-2) corresponding to the second ultrasound probe (100-2) and provided to hold the first ultrasound probe (100-1) and the second ultrasound probe (100-2);
**characterized in that** the controller (220) is configured to control the second light source (281-2) to irradiate light when proceeding from the first diagnosis step to the second diagnosis step.

2. The ultrasound imaging apparatus according to claim 1,
wherein the controller (220) is configured to turn on the first light source (281-1) or the second light source (281-2) based on the program.

3. The ultrasound imaging apparatus according to claim 1, further comprising:
an input unit (270) to receive a command from a user,
wherein the controller (220) is configured to store diagnosis contents of the first diagnosis step in the storage (290) when the user inputs a save command through the input unit (270) and to guide to replace with the second ultrasound probe (100-2) corresponding to the second diagnosis step.

4. The ultrasound imaging apparatus according to claim 3, wherein the input unit (270) receives information of the object from the user, and
wherein the controller (220) is configured to guide to replace the first ultrasound probe (100-1) with the second ultrasound probe (100-2) by using the program and the information of the object.

5. The ultrasound imaging apparatus according to claim 3, wherein the input unit (270) receives information related to the ultrasound image from the user, and
wherein the controller (220) is configured to guide to replace the first ultrasound probe (100-1) with the second ultrasound probe (100-2) by using the program and the information related to the ultrasound image.

6. The ultrasound imaging apparatus according to claim 1, wherein the storage (290) stores a set value of each of the first ultrasound probe (100-1) and the second ultrasound probe (100-2) when the ultrasound image is acquired, and
wherein the controller (220) is configured to guide to replace with the second ultrasound probe (100-2) corresponding to the second diagnosis step by applying the set value when changing from the first diagnosis step to the second diagnosis step based on the program.

7. The ultrasound imaging apparatus according to claim 1, wherein each of the first ultrasound probe (100-1) and the second ultrasound probe (100-2) is provided as one of a convex array probe, a linear array probe and a phased array probe.

8. The ultrasound imaging apparatus according to claim 1, wherein the second ultrasound probe (100-2) acquires an ultrasound image by using an ultrasound of a frequency band different from the first ultrasound probe (100-1).

9. A control method of an ultrasound imaging apparatus (10) according to claims 1 -8, comprising:
guiding a diagnosis sequence of a patient comprising a predetermined first diagnosis step and a second diagnosis step based on a program stored in a storage (290);
guiding (1001) to diagnose the patient using a first ultrasound probe (100-1) corresponding to the first diagnosis step;
guiding to replace with a second ultrasound probe (100-2) corresponding to the second diagnosis step when changing from the first diagnosis step to the second diagnosis step;
**characterized in that** the control method comprises controlling the second light source (281-2) corresponding to the second ultrasound probe (100-2) to irradiate light when proceeding from the first diagnosis step to the second diagnosis step.

10. The control method according to claim 9, further comprising:
turning on a first light source (281-1) corresponding to the first ultrasound probe (100-1) or the second light source (281-2) based on the program.

11. The control method according to claim 9, wherein the guiding to replace with the second ultrasound probe (100-2) comprises:
storing (1003) diagnosis contents of the first diagnosis step in the storage (290) when a user inputs (1002) a save command; and
guiding (1004) to replace with the second ultrasound probe (100-2) corresponding to the second diagnosis step.

12. The control method according to claim 11, further comprising:
receiving information of an object from the user,
wherein the guiding to replace with the second ultrasound probe (100-2) comprises:
guiding to replace the first ultrasound probe (100-1) with the second ultrasound probe (100-2) by using the program and the information of the object.

13. The control method according to claim 11, further comprising:
receiving information related to an ultrasound image from the user by an input unit (270),
wherein the guiding to replace with the second ultrasound probe (100-2) comprises:
guiding to replace the first ultrasound probe (100-1) with the second ultrasound probe (100-2) by using the program and the information related to the ultrasound image.

## Patentansprüche

1. Ultraschallbildgebungsvorrichtung (10), die Folgendes umfasst:
eine erste Ultraschallsonde (100-1), die dazu konfiguriert ist, durch Senden und Empfangen eines Ultraschallsignals ein Ultraschallbild eines Objekts zu erhalten;
eine zweite Ultraschallsonde (100-2), die als eine andere Art von Ultraschallsonde als die erste Ultraschallsonde (100-1) vorgesehen ist;
einen Speicher (290) zum Speichern eines Programms zum Anleiten einer Diagnosesequenz eines Patienten, die einen vorbestimmten ersten Diagnoseschritt und einen zweiten Diagnoseschritt umfasst;
eine Steuerung (220), die konfiguriert ist zum Anleiten, den Patienten entsprechend dem ersten Diagnoseschritt unter Verwendung der ersten Ultraschallsonde (100-1) zu diagnostizieren, und zum Anleiten, entsprechend dem zweiten Diagnoseschritt eine Ersetzung durch die zweite Ultraschallsonde (100-2) durchzuführen, wenn basierend auf dem Programm von dem ersten Diagnoseschritt zu dem zweiten Diagnoseschritt gewechselt wird; und
einen Halter (280), der eine erste Lichtquelle (281-1), die der ersten Ultraschallsonde (100-1) entspricht, und eine zweite Lichtquelle (281-2), die der zweiten Ultraschallsonde (100-2) entspricht, umfasst und vorgesehen ist zum Halten der ersten Ultraschallsonde (100-1) und der zweiten Ultraschallsonde (100-2);
**dadurch gekennzeichnet, dass** die Steuerung (220) dazu konfiguriert ist, die zweite Lichtquelle (281-2) so zu steuern, dass sie Licht abstrahlt, wenn von dem ersten Diagnoseschritt zu dem zweiten Diagnoseschritt übergegangen wird.

2. Ultraschallbildgebungsvorrichtung nach Anspruch 1,
wobei die Steuerung (220) dazu konfiguriert ist, basierend auf dem Programm die erste Lichtquelle (281-1) oder die zweite Lichtquelle (281-2) einzuschalten.

3. Ultraschallbildgebungsvorrichtung nach Anspruch 1, die ferner Folgendes umfasst:
eine Eingabeeinheit (270) zum Empfangen eines Befehls von einem Benutzer,
wobei die Steuerung (220) konfiguriert ist zum Speichern von Diagnoseinhalten des ersten Diagnoseschritts in dem Speicher (290), wenn der Benutzer einen Speicherbefehl über die Eingabeeinheit (270) eingibt, und zum Anleiten, entsprechend dem zweiten Diagnoseschritt eine Ersetzung durch die zweite Ultraschallsonde (100-2) durchzuführen.

4. Ultraschallbildgebungsvorrichtung nach Anspruch 3, wobei die Eingabeeinheit (270) von dem Benutzer Informationen über das Objekt empfängt und
wobei die Steuerung (220) konfiguriert ist zum Anleiten, die erste Ultraschallsonde (100-1) durch die zweite Ultraschallsonde (100-2) zu ersetzen, unter Verwendung des Programms und der Informationen über das Objekt.

5. Ultraschallbildgebungsvorrichtung nach Anspruch 3, wobei die Eingabeeinheit (270) Informationen in Bezug auf das Ultraschallbild von dem Benutzer empfängt, und
wobei die Steuerung (220) konfiguriert ist zum Anleiten, die erste Ultraschallsonde (100-1) durch die zweite Ultraschallsonde (100-2) zu ersetzen, unter Verwendung des Programms und der Informationen in Bezug auf das Ultraschallbild.

6. Ultraschallbildgebungsvorrichtung nach Anspruch 1, wobei der Speicher (290) einen eingestellten Wert sowohl der ersten Ultraschallsonde (100-1) als auch der zweiten Ultraschallsonde (100-2) speichert, wenn das Ultraschallbild erfasst wird, und
wobei die Steuerung (220) konfiguriert ist zum Anleiten, entsprechend dem zweiten Diagnoseschritt die erste Ultraschallsonde (100-1) durch die zweite Ultraschallsonde (100-2) zu ersetzen, indem der eingestellte Wert angewendet wird, wenn basierend auf dem Programm von dem ersten Diagnoseschritt zu dem zweiten Diagnoseschritt gewechselt wird.

7. Ultraschallbildgebungsvorrichtung nach Anspruch 1, wobei sowohl die erste Ultraschallsonde (100-1) als auch die zweite Ultraschallsonde (100-2) als eine von einer konvexen Array-Sonde, einer linearen Array-Sonde oder einer phasengesteuerten Array-Sonde vorgesehen ist.

8. Ultraschallbildgebungsvorrichtung nach Anspruch 1, wobei die zweite Ultraschallsonde (100-2) unter Verwendung eines Ultraschalls eines Frequenzbands, das sich von dem der ersten Ultraschallsonde (100-1) unterscheidet, ein Ultraschallbild erfasst.

9. Steuerungsverfahren einer Ultraschallbildgebungsvorrichtung (10) nach den Ansprüchen 1 bis 8, umfassend:
Anleiten einer Diagnosesequenz eines Patienten, die einen vorbestimmten ersten Diagnoseschritt und einen zweiten Diagnoseschritt umfasst, basierend auf einem in einem Speicher (290) gespeicherten Programm;
Anleiten (1001) zum Diagnostizieren des Patienten unter Verwendung einer ersten Ultraschallsonde (100-1) entsprechend dem ersten Diagnoseschritt,
Anleiten zum Durchführen einer Ersetzung durch eine zweite Ultraschallsonde (100-2) entsprechend dem zweiten Diagnoseschritt, wenn von dem ersten Diagnoseschritt zu dem zweiten Diagnoseschritt gewechselt wird;
**dadurch gekennzeichnet, dass** das Steuerungsverfahren das Steuern der zweiten Lichtquelle (281-2) umfasst, die der zweiten Ultraschallsonde (100-2) entspricht, um Licht abzustrahlen, wenn von dem ersten Diagnoseschritt zu dem zweiten Diagnoseschritt übergegangen wird.

10. Steuerungsverfahren nach Anspruch 9, das ferner Folgendes umfasst:
Einschalten einer der ersten Ultraschallsonde (100-1) entsprechenden ersten Lichtquelle (281-1) oder der zweiten Lichtquelle (281-2) basierend auf dem Programm.

11. Steuerungsverfahren nach Anspruch 9, wobei das Anleiten zum Durchführen einer Ersetzung durch die zweite Ultraschallsonde (100-2) Folgendes umfasst:
Speichern (1003) von Diagnoseinhalten des ersten Diagnoseschritts in dem Speicher (290), wenn ein Benutzer einen Speicherbefehl eingibt (1002); und
Anleiten (1004) zum Durchführen einer Ersetzung durch die zweite Ultraschallsonde (100-2) entsprechend dem zweiten Diagnoseschritt.

12. Steuerungsverfahren nach Anspruch 11, das ferner Folgendes umfasst:
Empfangen von Informationen über ein Objekt von dem Benutzer,
wobei das Anleiten zum Durchführen einer Ersetzung durch die zweite Ultraschallsonde (100-2) Folgendes umfasst:
Anleiten, die erste Ultraschallsonde (100-1) durch die zweite Ultraschallsonde (100-2) zu ersetzen unter Verwendung des Programms und der Informationen über das Objekt.

13. Steuerungsverfahren nach Anspruch 11, das ferner Folgendes umfasst:
Empfangen von Informationen in Bezug auf ein Ultraschallbild von dem Benutzer durch eine Eingabeeinheit (270),
wobei das Anleiten zum Durchführen einer Ersetzung durch die zweite Ultraschallsonde (100-2) Folgendes umfasst:
Anleiten, die erste Ultraschallsonde (100-1) durch die zweite Ultraschallsonde (100-2) zu ersetzen, unter Verwendung des Programms und der Informationen in Bezug auf das Ultraschallbild.

## Revendications

1. Appareil d'imagerie échographique (10), comprenant :
une première sonde échographique (100-1) conçue pour obtenir une image échographique d'un objet par émission et réception d'un signal ultrasonore ;
une deuxième sonde échographique (100-2) prévue sous la forme d'un type de sonde échographique différent de celui de la première sonde échographique (100-1) ;
un dispositif de stockage (290) destiné à stocker un programme visant à indiquer une séquence de diagnostic d'un patient comprenant une première étape de diagnostic prédéterminée et une deuxième étape de diagnostic ;
un organe de commande (220) conçu pour offrir une indication visant à diagnostiquer le patient au moyen de la première sonde échographique (100-1) dans le cadre de la première étape de diagnostic et pour offrir une indication visant à effectuer un remplacement par la deuxième sonde échographique (100-2) dans le cadre de la deuxième étape de diagnostic lors d'un changement de la première étape de diagnostic à la deuxième étape de diagnostic en fonction du programme ; et
un réceptacle (280) comprenant une première source de lumière (281-1) correspondant à la première sonde échographique (100-1) et une deuxième source de lumière (281-2) correspondant à la deuxième sonde échographique (100-2) et prévu pour recevoir la première sonde échographique (100-1) et la deuxième sonde échographique (100-2) ;
**caractérisé en ce que** l'organe de commande (220) est conçu pour commander la deuxième source de lumière (281-2) de façon qu'elle émette de la lumière lors du passage de la première étape de diagnostic à la deuxième étape de diagnostic.

2. Appareil d'imagerie échographique selon la revendication 1,
dans lequel l'organe de commande (220) est conçu pour activer la première source de lumière (281-1) ou la deuxième source de lumière (281-2) en fonction du programme.

3. Appareil d'imagerie échographique selon la revendication 1, comprenant en outre :
une unité d'entrée (270) destinée à recevoir une commande en provenance d'un utilisateur ;
ledit organe de commande (220) étant conçu pour stocker du contenu de diagnostic de la première étape de diagnostic dans le dispositif de stockage (290) lorsque l'utilisateur saisit une commande d'enregistrement par le biais de l'unité d'entrée (270) et pour offrir une indication visant à effectuer un remplacement par la deuxième sonde échographique (100-2) dans le cadre de la deuxième étape de diagnostic.

4. Appareil d'imagerie échographique selon la revendication 3, dans lequel l'unité d'entrée (270) reçoit des informations relatives à l'objet en provenance de l'utilisateur, et
dans lequel l'organe de commande (220) est conçu pour offrir une indication visant à effectuer un remplacement de la première sonde échographique (100-1) par la deuxième sonde échographique (100-2) au moyen du programme et des informations relatives à l'objet.

5. Appareil d'imagerie échographique selon la revendication 3, dans lequel l'unité d'entrée (270) reçoit des informations en lien avec l'image échographique en provenance de l'utilisateur, et
dans lequel l'organe de commande (220) est conçu pour offrir une indication visant à effectuer un remplacement de la première sonde échographique (100-1) par la deuxième sonde échographique (100-2) au moyen du programme et des informations en lien avec l'image échographique.

6. Appareil d'imagerie échographique selon la revendication 1, dans lequel le dispositif de stockage (290) stocke une valeur définie de la première sonde échographique (100-1) et de la deuxième sonde échographique (100-2) lorsque l'image échographique est acquise, et
dans lequel l'organe de commande (220) est conçu pour offrir une indication visant à effectuer un remplacement par la deuxième sonde échographique (100-2) dans le cadre de la deuxième étape de diagnostic en appliquant la valeur définie lors du changement de la première étape de diagnostic à la deuxième étape de diagnostic en fonction du programme.

7. Appareil d'imagerie échographique selon la revendication 1, dans lequel la première sonde échographique (100-1) et la deuxième sonde échographique (100-2) sont chacune prévues sous la forme d'une sonde à réseau convexe, d'une sonde à réseau linéaire ou d'une sonde à réseau à commande de phase.

8. Appareil d'imagerie échographique selon la revendication 1, dans lequel la deuxième sonde échographique (100-2) acquiert une image échographique à l'aide d'ultrasons dont la bande de fréquence est différente de celle de la première sonde échographique (100-1).

9. Procédé de commande d'un appareil d'imagerie échographique (10) selon les revendications 1 à 8, comprenant :
l'indication d'une séquence de diagnostic d'un patient comprenant une première étape de diagnostic prédéterminée et une deuxième étape de diagnostic en fonction d'un programme stocké dans un dispositif de stockage (290) ;
une indication (1001) visant à diagnostiquer le patient au moyen d'une première sonde échographique (100-1) dans le cadre de la première étape de diagnostic ;
une indication visant à effectuer un remplacement par une deuxième sonde échographique (100-2) dans le cadre de la deuxième étape de diagnostic lors du changement de la première étape de diagnostic à la deuxième étape de diagnostic ;
le procédé de commande étant **caractérisé en ce qu'**il comprend la commande de la deuxième source de lumière (281-2) correspondant à la deuxième sonde échographique (100-2) de façon qu'elle émette de la lumière lors du passage de la première étape de diagnostic à la deuxième étape de diagnostic.

10. Procédé de commande selon la revendication 9, comprenant en outre :
l'activation d'une première source de lumière (281-1) correspondant à la première sonde échographique (100-1) ou de la deuxième source de lumière (281-2) en fonction du programme.

11. Procédé de commande selon la revendication 9, dans lequel l'indication visant à effectuer un remplacement par la deuxième sonde échographique (100-2) comprend :
le stockage (1003) d'un contenu de diagnostic de la première étape de diagnostic dans le dispositif de stockage (290) lorsqu'un utilisateur saisit (1002) une commande d'enregistrement, et
une indication (1004) visant à effectuer un remplacement par la deuxième sonde échographique (100-2) dans le cadre de la deuxième étape de diagnostic.

12. Procédé de commande selon la revendication 11, comprenant en outre :
la réception d'informations concernant un objet en provenance de l'utilisateur ;
ladite indication visant à effectuer un remplacement par la deuxième sonde échographique (100-2) comprenant :
une indication visant à effectuer un remplacement de la première sonde échographique (100-1) par la deuxième sonde échographique (100-2) au moyen du programme et des informations concernant l'objet.

13. Procédé de commande selon la revendication 11, comprenant en outre :
la réception d'informations en lien avec une image échographique en provenance de l'utilisateur par une unité d'entrée (270) ;
ladite indication visant à effectuer un remplacement par la deuxième sonde échographique (100-2) comprenant :
une indication visant à effectuer un remplacement de la première sonde échographique (100-1) par la deuxième sonde échographique (100-2) au moyen du programme et des informations en lien avec l'image échographique.
